# EUROPEAN PATENT APPLICATION

(11) **EP 4 534 053 A1**
(43) Date of publication of application: **09.04.2025**
(21) Application number: 24202164.0
(22) Date of filing: 24.09.2024
(51) Int. Cl.: A61F 9/00, A61F 2/02

(54) **MEDICAL DEVICE**

(30) Priority: 06.10.2023 SE 2351151
(71) Applicant: Berggren, Per-Olof, 169 55 Solna (SE); Herland, Anna, 18733 Täby (SE); Kavand, Hanie, 11759 Stockholm (SE); Köhler, Martin, 14140 Huddinge (SE); Van Der Wijngaart, Wouter, 192 51 Sollentuna (SE)
(72) Inventor: Berggren, Per-Olof, 169 55 Solna (SE); Herland, Anna, 18733 Täby (SE); Kavand, Hanie, 11759 Stockholm (SE); Köhler, Martin, 14140 Huddinge (SE); Van Der Wijngaart, Wouter, 192 51 Sollentuna (SE)
(74) Representative: Potter Clarkson

(57) **Abstract**

There is provided an implantable medical device comprising a modality delivery structure (20), wherein the modality delivery structure (20) includes:
a confinement volume (24) for confining a modality element (22), wherein the confinement volume (24) is configured to, in use, enable at least partial interaction of the confined modality element (22) with the environment outside of the confinement volume (24);
an access element (26) that is mechanically openable and closeable to control access into the confinement volume (24); and
an anchor portion (28) for mechanical anchoring within a human or animal body or body part.

## Description

This invention relates to an implantable medical device, a method of configuring an implantable medical device and a method of implanting an implantable medical device.

It is known to transplant biocompatible medical devices into a human or animal body.

According to a first aspect of the invention, there is provided an implantable medical device comprising a modality delivery structure, wherein the modality delivery structure includes:
a confinement volume for confining a modality element, wherein the confinement volume is configured to, in use, enable at least partial interaction of the confined modality element with the environment outside of the confinement volume;
an access element that is mechanically openable and closeable to control access into the confinement volume; and
an anchor portion for mechanical anchoring within a human or animal body or body part.

For the purposes of this specification, the human or animal body or body part is intended to cover all parts that make up a human or animal organism.

The confinement volume may be configured to, in use, enable at least partial vascularisation of the confined modality element with the environment outside of the confinement volume.

The confinement volume may be configured to, in use, enable at least partial release of the confined modality element out of the confinement volume.

In embodiments of the invention, the confinement volume may be a confinement cage.

In further embodiments of the invention, the access element may include at least one flap member that is mechanically openable and closeable to control access into the confinement volume. In such embodiments, the access element may include a pair of opposing flap members that is mechanically openable and closeable to control access into the confinement volume. The access element may be movable to a first position to permit access into the confinement volume, and the access element may be movable to a second position to restrict access into the confinement volume. The access element may be configured to automatically return to the second position from the first position upon removal of a force applied on the access element. The access element may be made of a resilient material, e.g. a polymeric material. A non-limiting example of the polymeric material is a photoresin material.

The anchor portion may be configured as an anchor portion for sutureless mechanical anchoring within the human or animal body or body part. The anchor portion may be configured as a non-penetrative anchor portion for mechanical anchoring within the human or animal body or body part.

The anchor portion may be shaped as a wedge. The modality delivery structure may be shaped as a wedge.

The implantable medical device is especially useful for fixation in organs or tissue that, due to their size, shape and/or location, have limited space for implanting the implantable medical device or have particular geometry that corresponds to the shape of the implantable medical device.

The confinement volume may be arranged between first and second layers that are angled with respect to each other. The first and second layers may be arranged at an acute angle with respect to each other.

The access element may be formed in the first layer. Pores may be formed in the modality delivery structure. In particular, pores may be formed in the first layer, the second layer or any structural element interconnecting the first and second layers.

The implantable medical device may be an intraocular implant. For example, the anchor portion may be configured for mechanical anchoring within an iridocorneal angle of the eye. In particular, the anchor portion may be shaped to correspond to geometry of the iridocorneal angle of the eye.

The modality element may include, but is not limited to, tissue, a pancreatic islet, a therapeutic modality element, a drug and/or a medicament.

According to a second aspect of the invention, there is provided a method of configuring an implantable medical device according to any one of the first aspect of the invention and its embodiments, wherein the method comprises the steps of opening the access element and inserting a modality element into the confinement volume.

The method may include the step of using a manipulator tool to hold the modality element, open the access element and insert the modality element into the confinement volume.

According to a second aspect of the invention, there is provided a method of implanting an implantable medical device according to any one of the first aspect of the invention, wherein the method comprises the step of implanting the implantable medical device into a human or animal body or body part.

The method may include the step of inserting the anchor portion of the modality delivery structure into an eye. The method may include the step of inserting the anchor portion of the modality delivery structure into an iridocorneal angle of an eye.

The steps of the methods of the second and third aspects of the invention may be combined.

The use of the terms "first" and "second", and the like, in this patent specification is merely intended to help distinguish between similar features, and is not intended to indicate the relative importance of one feature over another feature, unless otherwise specified.

Within the scope of this application it is expressly intended that the various aspects, embodiments, examples and alternatives set out in the preceding paragraphs, and the claims and/or the following description and drawings, and in particular the individual features thereof, may be taken independently or in any combination. That is, all embodiments and all features of any embodiment can be combined in any way and/or combination, unless such features are incompatible. The applicant reserves the right to change any originally filed claim or file any new claim accordingly, including the right to amend any originally filed claim to depend from and/or incorporate any feature of any other claim although not originally claimed in that manner.

Preferred embodiments of the invention will now be described, by way of non-limiting examples, with reference to the accompanying drawings in which:
Figure 1 shows a schematic overview of localization, transplantation and application of pancreatic islet biohybrid microstructures;
Figure 2 shows transplantation and in vivo functionality of a wedge microstructure;
Figure 3 shows foreign body reaction characterization of microstructure explants;
Figure 4 shows biohybrid microstructure vascularization;
Figure 5 shows a cylinder microstructure design and islet localization procedure;
Figure 6 shows optical characterization of materials for 3D printing;
Figure 7 shows an overview of pancreatic islets and validation of biohybrid assembly and transplantation;
Figure 8 shows vascularization of cylinder microstructures; and
Figure 9 shows mouse eyes after transplantation.

The figures are not necessarily to scale, and certain features and certain views of the figures may be shown exaggerated in scale or in schematic form in the interests of clarity and conciseness.

The following embodiments of the invention are described with reference to an intraocular implant and the transplantation and vascularization of a pancreatic islet in the anterior chamber of an eye. The following embodiments of the invention apply mutatis mutandis to the use of the implantation medical device in other parts of a human or animal body or body part, the transplantation/implantation of other types of tissues, and the delivery of other types of modality elements such as a therapeutic modality element, a drug and a medicament, including small molecules.

Hybridizing biological cells with man-made sensors enables the detection of a wide range of weak physiological responses with high specificity. The anterior chamber of the eye (ACE) is an ideal transplantation site due to its ocular immune privilege and optical transparency, which enables superior non-invasive longitudinal analyses of cells and microtissues.

Engraftment of biohybrid microstructures in the ACE might, however, be affected by the pupillary response and dynamics. In the invention, sutureless transplantation of biohybrid microstructures 3D printed in IP-Visio containing a precisely localized pancreatic islet to the ACE of mice is presented. The biohybrid microstructures allow mechanical fixation in the ACE, independent of the iris dynamics. After transplantation, the islets in the microstructures successfully sustain their functionality for over 20 weeks and become vascularized despite physical separation from the vessel source (iris) and immersion in a low-viscous liquid (aqueous humour) with continuous circulation and clearance. This approach opens up new perspectives in biohybrid microtissue transplantation in the ACE, advancing monitoring of microtissue-host interactions, disease modelling, treatment outcomes, and vascularization in engineered tissues.

### Introduction

The development of implantable biomedical sensors is moving towards real-time in vivo screening. Sensors based on electrical, mechanical or optical modules have been developed for real-time measuring of arterial pressure and pulse rate, neuronal activities, glucose monitoring and intraocular pressure (IOP). However, biosensors composed of living cells as a part of their sensing element, known as biohybrid sensors, are considered superior to biosensors that exclusively depend on physicochemical changes near the sensor. This is because the biological unit in biohybrid sensors can sense and respond to a wider range of weak physiological stimuli with higher specificity and can integrate with the host through vascularization to create a more physiological connection to the external evaluation units. Biohybrid sensors show potential for a wide range of applications such as odorant and taste sensing, directional chemical source detection, infrared detection and drug evaluation. However, to extend these applications to in vivo microenvironments, new strategies are needed that can meet the requirements of the host tissue, the biological sensing, and the non-biological readout units.

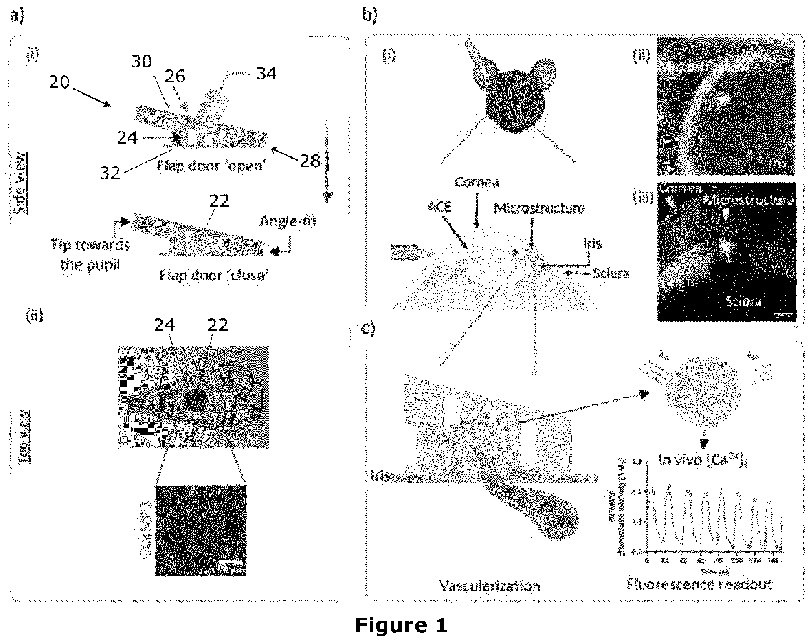

The inventors have developed an implantable medical device comprising a modality delivery microstructure 20 (hereon referred to as "microstructure") that can both securely hold a microtissue 22 in a specific location within the ACE and promote tissue engraftment (as shown in Figure 1).

Generally the microstructure 20 includes:
- a confinement cage 24 for confining the microtissue 22, wherein the confinement cage 24 is configured to, in use, enable at least partial interaction of the confined microtissue 22 with the environment outside of the confinement cage 24;
- an access element that is mechanically openable and closeable to control access into the confinement cage 24. In this embodiment, the access element includes a pair of opposing flap doors 26 that is mechanically openable and closeable to control access into the confinement cage 24. In use the flap doors 26 are movable to a first open position to permit access into the confinement cage 24 and thereby permit insertion of the microtissue 22 into the confinement cage 24, and the flap doors 26 are movable to a second closed position to restrict access into the confinement cage 24 and thereby confine the microtissue 22 inside the confinement cage 24. The flap doors 26 may be made of a resilient material so that it is configured to automatically return to the second closed position from the first open position upon removal of a force applied on the flap doors 26; and
- an anchor portion 28 that is configured as a non-penetrative anchor portion 28 for sutureless mechanical anchoring within the ACE.

Pancreatic islets are exemplarily chosen as the model microtissue due to their importance and widespread use in islet allotransplantation as a curative therapy for type 1 diabetes and their potential for glucose-sensing applications. Several locations may be used as islet transplantation sites in rodents and higher mammalian models, including the subcutaneous space, intra-hepatic infusion, spleen, kidney capsule, and ACE. The ACE is an ideal transplantation site due to its immune-privileged status and a high degree of vascularization, which provides an excellent environment for engraftment and allows for tracking host-transplant interactions.

While hybrid scaffolds have been engineered for subcutaneous islet transplantation and sensors have been designed to measure IOP in the ACE, no study to the inventors' knowledge has examined the engraftment of biohybrid microstructures in this location. Nano/microencapsulation of microtissues in polymer membranes has been widely used although they often create complexities for metabolite and growth factor diffusion, hinder subsequent engraftment (intra-microtissue vessel development) and are less flexible to be tailored for various applications. In contrast, the invention enables assessment of engraftment in a micromechanical structure where cells are in direct contact with the ACE microenvironment, allowing for vascular engraftment without altering the structure mechanically.

The inventors have investigated the engraftment of biohybrid microstructures in the ACE and to provide insights for developing future biohybrid platforms, such as sensors, designed for this region. To successfully integrate biohybrid devices with host biology, three requirements must be met: 1\) protection of the microtissue 22 during handling, 2) preservation of the functionality of the biological unit, and 3) promotion of integration through vascularization.

Figure 1 shows a schematic overview of localization, transplantation and application of pancreatic islet biohybrid microstructures, as follows:
a) The microstructures 20 are designed based on the anatomy of the mouse's ACE and fabricated via two-photon polymerization (TPP) 3D printing.
   i. The microtissue 22 (in this case, a pancreatic islet) is placed in the confinement cage 24 of the microstructure 20 using a micromanipulator 34 and mechanically confined by the flap doors 26 that opens during the placement by the gentle pressure of the cannula tip and closes after cannula tip retrieval.
   ii. Photograph of a microstructure 20 with a localized islet inside. (Scale bar = 200 µm). The magnified confocal image shows the fluorescence intensity difference between the microstructure and the islet expressing the GCaMP3 biosensor protein.
b) The microstructures 20 are transplanted into the ACE.
   i. By way of sutureless transplantation to the ACE, the microstructure 20 is placed at the iridocorneal angle (located on the circumference of the ACE) through an incision in the cornea.
   ii. Photograph of a mouse eye with a transplanted microstructure 20. Arrowheads denote the microstructure (yellow), islet (white), and iris (magenta).
   iii. Confocal backscatter image (maximum projection) of a transplanted eye. Corneal transparency allows longitudinal imaging. Arrowheads denote the cornea (cyan), microstructure (yellow), iris (magenta), and sclera (white). (Scale bar = 200 µm).
c) Schematic illustration of the engraftment to the host tissue. Ca²⁺ imaging is used to track the Ca²⁺ oscillations, as a functional state tracer of the transplants, in vivo.

### Results and Discussion

### Design and fabrication of the microstructure for microtissue transplantation in the ACE

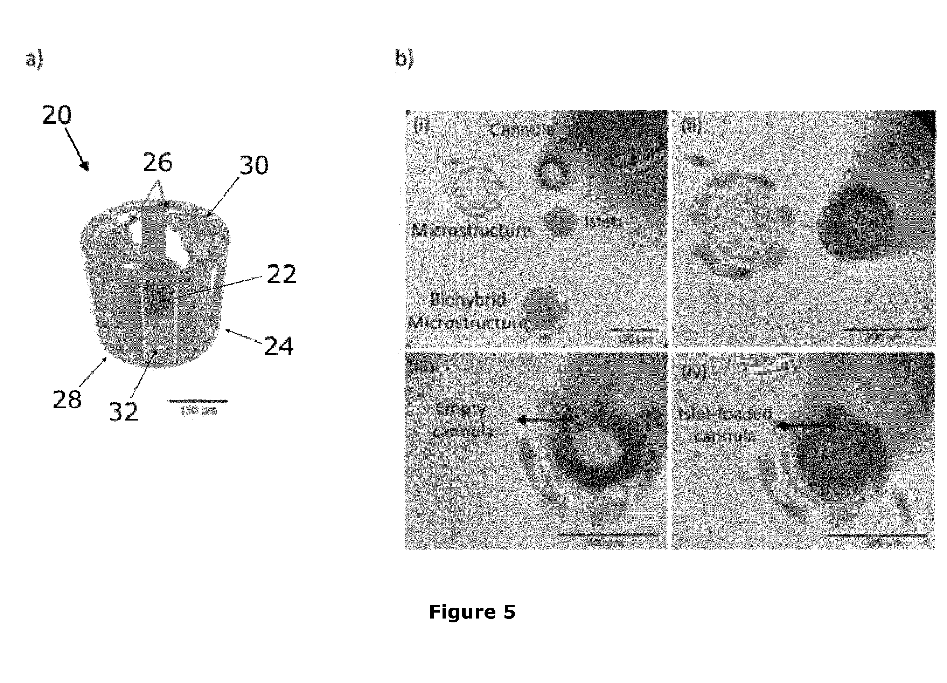

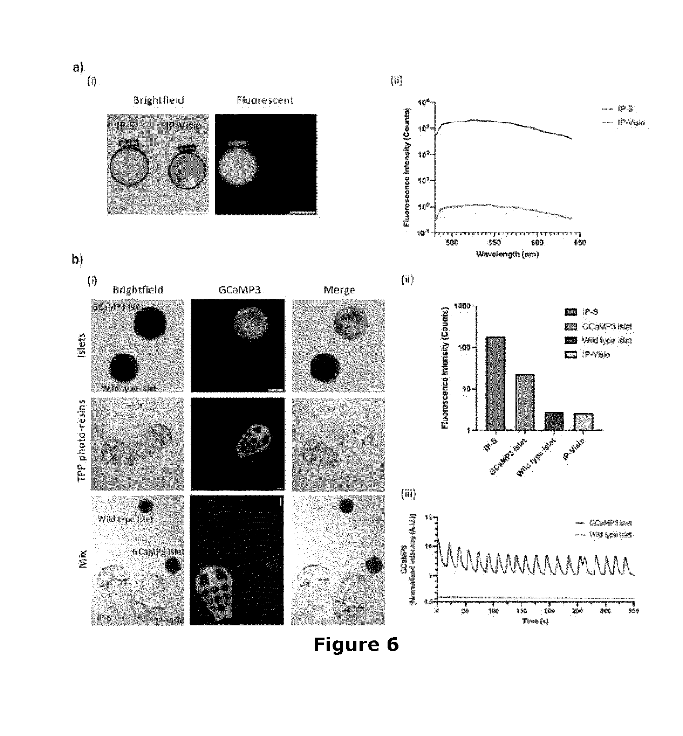

To ensure successful sutureless transplantation of microstructures 20 to the mouse ACE, the inventors designed two different microstructures 20, named "cylinder" and "wedge" after their shapes, based on the mouse eye's anatomy and sutureless transplantation requirements. Two-photon polymerization (TPP) technique was used to 3D print these microstructures 20 using IP-Visio photoresin, as illustrated in Figure 5 (cylinder) and Figure 1 (wedge). Two different materials were investigated for the structural component of the microstructures 20, IP-Visio and IP-S. IP-Visio was selected due to its suitable optical properties (such as transparency and autofluorescence) and mechanical properties (as shown in Figure 6). To securely contain the microtissue 22 inside the microstructure 20 during transplantation, the flap doors 26 of the microstructure 20 can be mechanically opened during microtissue placement using a cannula and then mechanically closed to mechanically confine the microtissue 22 after placement, as shown in Figures 1 and 6. This feature eliminates the need for additional extracellular matrix-like adhesives, such as collagen hydrogels or Matrigel, and avoids related complexities such as tissue/organoid functionality, optical limitations, or diffusion limitation.

The cylinder has a base diameter of 300 µm and height of 240 µm, consisting of a first top layer 30 with flap doors 26 for microtissue placement, a second base layer 32 with a diameter of 20 µm macropores for media exchange, and a plurality of pillars spaced at 70 µm intervals to connect the top and base layers so as to form the confinement cage 24, as depicted in Figure 5.

The wedge is designed to fit and mechanically wedge into the iridocorneal angle of the ACE (Figure 1) located on the circumference of the ACE, intermediating the sides of the base of the iris, cornea, sclera and the anterior surface of the ciliary body. In the wedge, the confinement cage 24 is formed by a plurality of pillars arranged between a first top layer 30 and a second base layer 32 that are arranged at an acute angle with respect to each other. The flap doors 26 are formed in the top layer 30, while pores may be formed in the base layer 32. This location is strategically chosen due to its minimal iris dynamics, which minimizes the risk of continuous displacement during grafting. The rear of the wedge is designed to fit the angle, with the tip oriented towards the pupil. The wedge design also incorporates the flap doors 26 for easy placement and mechanical confinement of the microtissue 22. Pores or voids (e.g. with sizes of 40-632 µm) are incorporated into the cylinder and wedge microstructures 20 to ensure optimal functionality and long-term use. Large pore sizes generally promote more extensive and sustained vascular ingrowth compared to smaller pore sizes. Further to their role in promoting vascularization of the confined microtissue 22, they prevent obstruction of aqueous humor drainage via the Schlemm's canal, which if blocked, could lead to elevated IOP.

### Islet isolation, localization in the microstructure, and in vitro functionality

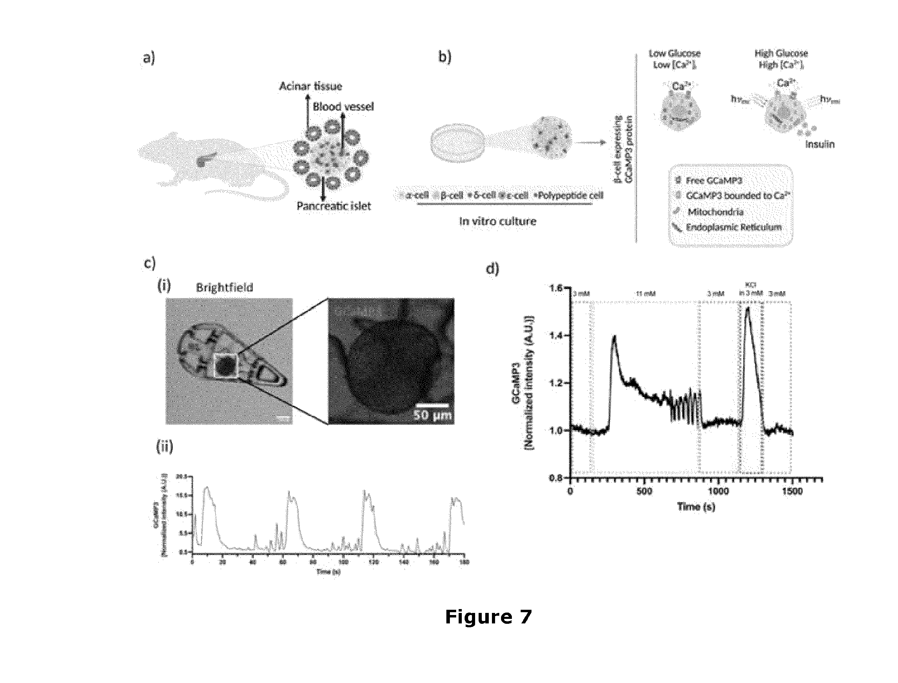

Pancreatic islets are chosen as the microtissue model. Specifically either wild-type islets or islets that expressed the GCaMP3 Ca²⁺ biosensor in their β-cells are used (see Figure 7a,b). After islet isolation and a day in culture, the islets are placed inside the microstructures 20 using a cannula and a micromanipulator (Figure 1a). Islet functionality and absence of mechanical damage are confirmed by examining their [Ca²⁺]ᵢ (cytoplasmic free Ca²⁺ concentration) dynamics in vitro (Figure 7c), where islets in buffer media containing 11 mM glucose presented functional [Ca²⁺]ᵢ oscillations. The in vitro biocompatibility of the IP-Visio photoresin is assessed by analyzing the islet glucose stimulation response one week after placement in the microstructure 20 (Figure 7d). The fluctuations of [Ca²⁺]ᵢ in response to different media containing 3 mM (basal) and 11 mM (high) glucose and 3 mM glucose with 25 mM KCI indicated a functional β-cell population (Figure 7d). KCl promotes the β-cell to depolarization, leading to a significant influx of Ca²⁺. In vitro results confirmed that the material and localization procedure had no evident adverse effect on β-cell functionality. Results show that the lower fluorescence intensity of IP-Visio compared to islets expressing GCaMP3 makes it a suitable material combination for evaluating [Ca²⁺]ᵢ dynamics.

### Transplantation and localization in the ACE

The microstructures 20 were transplanted into the ACE of mice using a self-healing cannula placement procedure (detailed in the Experimental Section). Donor islets were used 2-4 days after isolation, utilizing the temporary availability of intra-islet donor endothelial cells for elevated revascularization. Each eye received one microstructure 20. As a control, microstructures 20 without islets and with bare islets were transplanted.

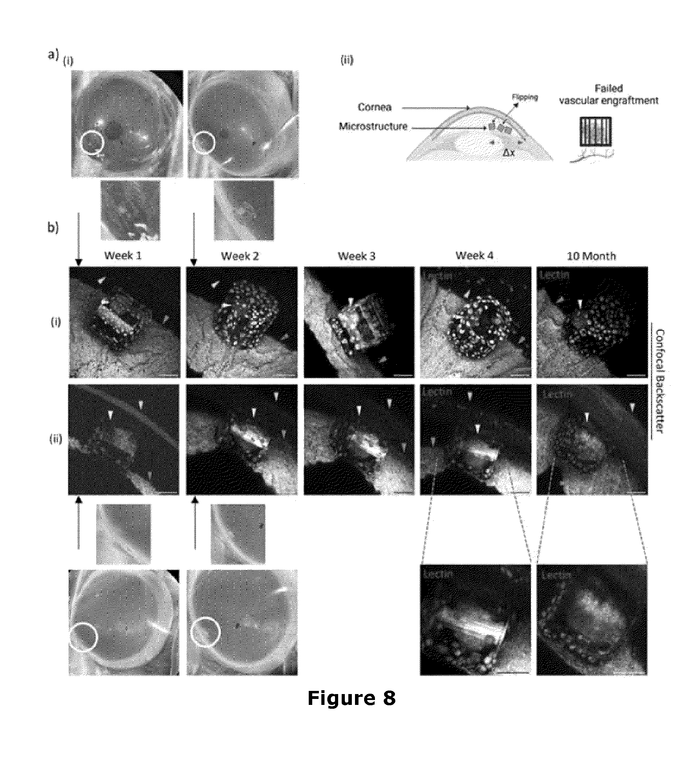

Initially cylinder microstructures 20 carrying non-sensor-expressing islets were transplanted into seven eyes respectively. The size and geometry of the microstructures 20 facilitated the transplantation procedure. The mice were evaluated up to 10 months after transplantation, and the microstructures 20 were examined for vascularization at several time points (Figure 8). The eyes showed normal morphology, no visible damage to the cornea was observed from the transplantation procedure, and the insertion incision was healed after a few days. Five structures were observed to be constantly localized on the iris; however their orientation differed at each evaluation time point. Vascularization in these samples were not detected on evaluations after four weeks or ten months post-transplantation (vascular engraftment is detailed in the section 'Vascular engraftment') (Figure 8a,b-i). In comparison, engraftment was observed in two structures that were physically trapped at the angle during evaluation four weeks post-transplantation (in contact with the cornea and the iris), and these structures did not show changes in localization or orientation during the experimental period (Figure 8b-ii).

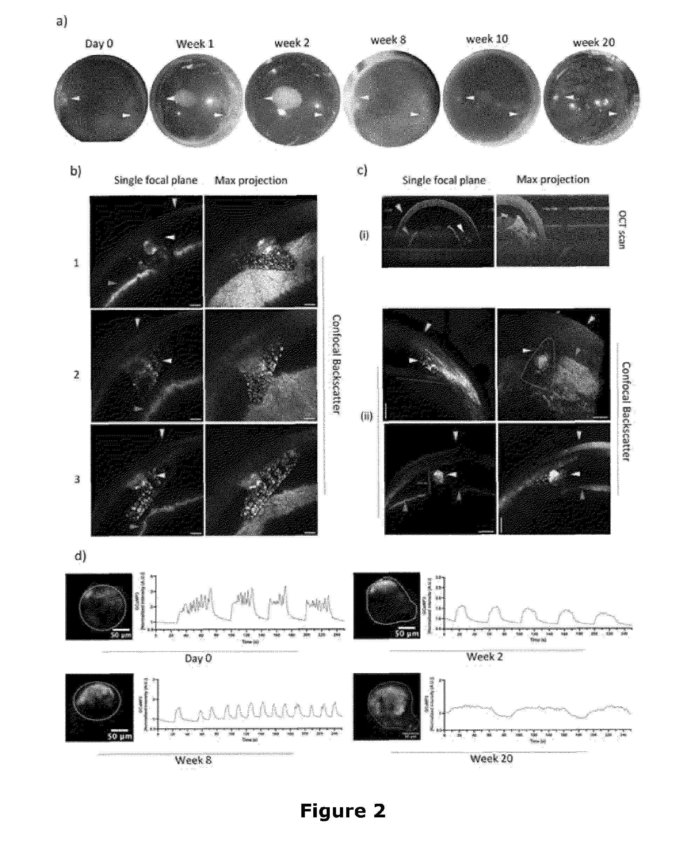

Figure 2 shows transplantation and in vivo functionality of a wedge microstructure 20.
a) Photographs of a transplanted eye at different time points show the microstructure 20 firmly localized at the transplantation site. The white arrowhead denotes the transplantation incision. The yellow arrowhead points to the microstructure 20. The white patches seen in the images of week 1 and week 2 are temporary and frequently observed after the specific anaesthesia used.
b) Confocal backscatter images from different microstructures (1, 2 and 3) after transplantation. Arrowheads denote the cornea (cyan), iris (magenta), and microstructure 20 (yellow). (Scale bar = 100 µm)
c) Images
   i. An OCT scan of an eye transplanted with a microstructure 20 showing intact morphology of the cornea after transplantation.
   ii. Confocal backscatter images of the microstructures 20 at the end of the experiment. (Scale bar = 200 µm). Confocal images are presented as maximum intensity projections in the XY plane.
d) Longitudinal intravital [Ca²⁺]ᵢ imaging of an islet in a microstructure 20 by confocal microscopy at indicated time points after transplantation. GCaMP3 fluorescence (green) was optically measured and plotted in the respective graphs. Images and graphs represent one sample over the experimental period.

The challenge of grafting on the iris can be linked to the natural iris dynamics. To resolve this challenge, the inventors designed the wedge microstructure geometry to fit the angle dimensions of the ACE. The intimate surface interaction between the microstructure 20 and the ACE boundary (iris, angle, and cornea) mechanically anchors the microstructure 20 in the ACE. Eleven wedge microstructures 20 were transplanted and evaluated for up to 20 weeks. The wedge dimensions allowed smooth insertion through the incision while preventing structural movement related to the iris dynamics (Figure 2a). Wedge rotation or displacement relative to the eye were not observed during the experimental period, thus indicating that the impact of iris dynamics on the microstructures 20 were greatly minimised. The orientation of the implanted wedge microstructures 20 is determined by their initial orientation during transplantation and is not affected by iris dynamics. It is envisaged that better control over the orientation of the implanted wedge microstructures 20 can be achieved through additional enhancements, such as better tools, better surgical insertion procedures or using materials that ensure consistent orientation when transplanted.

Optical coherence tomography (OCT) scans provided confirmation that the cornea remained intact after the transplantation (Figure 2c-i) and the localization of the microstructures 20 at the terminal time point (Figure 2c-ii). It should be noted that the microstructure 20 (IP-Visio) is not visible due to its transparency at the acquisition wavelength of the OCT scanner (840 nm). Nevertheless, the islet and the foreign body reaction (FBR) cells attached to the microstructure 20 were visible in the OCT scans.

### In vivo functionality

Presence of [Ca²⁺]ᵢ oscillations after transplantation is a critical indicator of success of the transplantation procedure. In vivo imaging analysis showed [Ca²⁺]ᵢ oscillations on the day of transplantation and two, eight and 20 weeks after transplantation, as depicted in Figure 2d. Notably, the mice were not subjected to fasting to maintain low blood glucose levels or injected with glucose to elevate their blood glucose levels during Ca²⁺ imaging. Therefore, a variation in frequency and magnitude of [Ca²⁺]ᵢ oscillations on the separate days of recording was expected and observed. Nevertheless, the consistent occurrence of [Ca²⁺]ᵢ oscillations throughout the study period suggests successful graft integration with the host. This was based on successful recording of [Ca²⁺]_{I} oscillations in 10 out of 11 samples, indicating in vivo functionality of islet β-cells.

### Foreign body reaction (FBR)

Less than 24 hours after transplantation in a mouse model recognized for inducing strong FBR, an accumulation of cells on the microstructure 20 was observed, implying FBR (Figure 8b). The sparse monolayer coverage of circular cells on the microstructures 20 remained consistent between different in vivo assessment time points, as observed by intravital microscopy.

After graft retrieval at ten months (cylinder) and 20 weeks (wedge) post-transplantation, most of the cells covering the microstructure 20 had brownish compartments inside their cytoplasm, indicating the presence of macrophages with phagosomes (Figure 7a). The presence of macrophages and foreign body giant cells (FBGCs) as markers of inflammation on the transplanted structures were investigated. This investigation included observation for cells staining positive for F4/80 (the most specific marker for murine macrophages), CD11b (a marker for murine dendritic cells), and α-SMA (a myofibroblast marker) with a round/oval, round/spread, and spindle shape morphology, respectively (Figure 7b,c). Multinucleated FBGCs with filopodia were identified by immunostaining for intracellular vimentin, a highly abundant cytoplasmic intermediate filament protein expressed in inflammatory macrophages (Figure 7d). Since pro- and anti-inflammatory cytokines stimulate macrophages to secrete vimentin, the samples were also stained for extracellular vimentin (Figure 7e). No evidence of thick extracellular matrix (ECM) or accumulation of multi-layered cells on the surface of the structure was found. This could be attributed to the immune-privileged microenvironment of the ACE or a minimized immunogenicity of IP-Visio. In some samples, FBR cells were observed to be partially in contact with the islet (Figure 7f). However, a specific alteration in β-cell function was not observed.

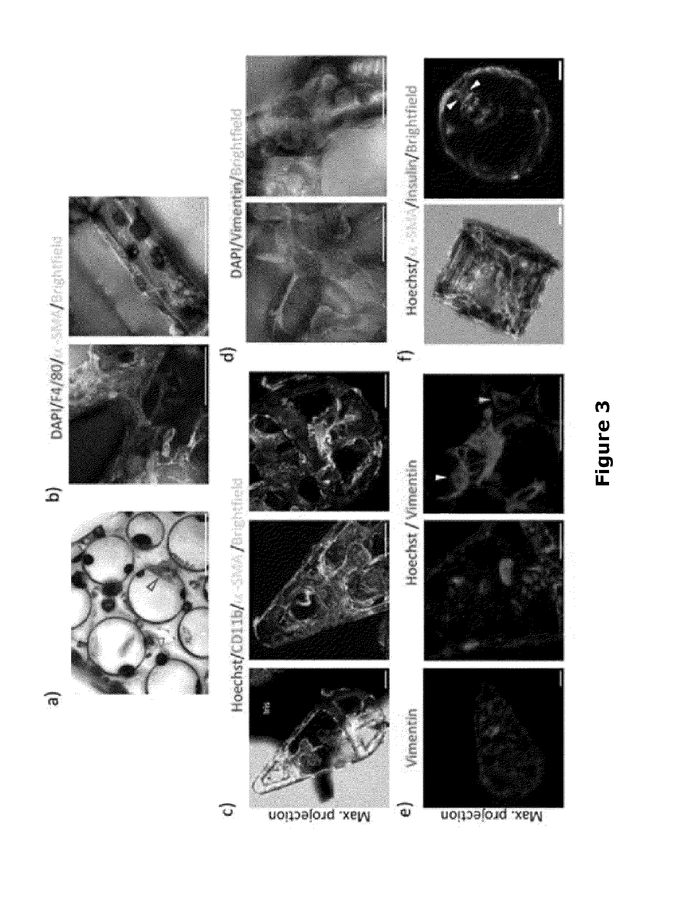

Figure 3 shows foreign body reaction characterization of microstructure explants retrieved after 10 months (cylinder) and 20 weeks (wedge) of transplantation. All scale bars represent 100 µm unless stated otherwise.
a) Optical image of a microstructure explant covered by cells including a high concentration of phagosome (green arrowhead), moderate cytoplasmic phagosomes (blue arrowhead), and residual endothelial cells (red arrowhead) with non-cytoplasmic phagosomes.
b) Cell characterization by confocal microscopy after immuno-fluorescently labeling for F4/80, a macrophage marker (red), α-SMA, the myofibroblast marker (green), CD11b, a macrophage marker (magenta), and nuclei (blue).
c) Cell characterization by confocal microscopy after immuno-fluorescently labeling for F4/80, a macrophage marker (red), α-SMA, the myofibroblast marker (green), CD11b, a macrophage marker (magenta), and nuclei (blue).
d) Immunofluorescence images of cell cytoskeletal morphology (intermediate filaments) using immunostaining for vimentin (red). Foreign body giant cells (FBGCs) are identified by the appearance of multiple nuclei. (Scale bar = 50 µm).
e) Immunostaining images of extracellular vimentin secreted by the cells represented in maximum projection. The white arrowhead points to FBGCs and the green arrowhead points to a cell with incomplete imaging stacks showing the stained cells' cross-sectional view.
f) Confocal images of a microstructure 20 (side and top cross-section views) with an islet immunolabeled for insulin (magenta) and α-SMA (green). White arrowhead points to the interaction site between the islet and the α-SMA⁺ cells. Vascular engraftment

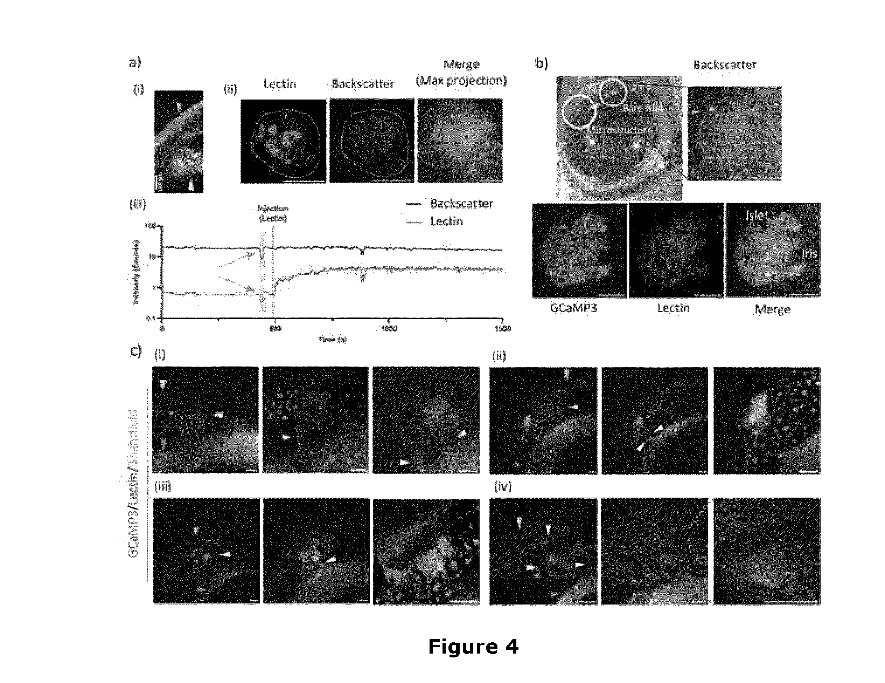

Figure 4 shows biohybrid microstructure vascularization. All scale bars represent 100 µm unless stated otherwise. Arrowheads denote cornea (cyan), microstructure 20 (yellow), iris (magenta), and vessels (white).
a) Images
   i. Confocal backscatter image of a transplanted microstructure 20.
   ii. Intravital fluorescence image of a microstructure 20 twenty weeks after transplantation. The fluorescent dye, DyLight-Lectin, perfused blood vessels and was administered through tail vein injection. (Scale bar = 50 µm).
   iii. The time-dependent increase in fluorescence signal plotted in this image indicates successful vascularization of the islet. The arrows point to the animal's transient movement (motion artifact) during preparation for injection, while the dotted line marks the time when the dye reaches the intra-islet vessels via the bloodstream. Movements result in a loss of focus and changes in the signal that could also be seen in the backscatter/reflection channel.
b) Photograph of an eye transplanted with a microstructure 20 and a bare islet as a positive control for the engraftment procedure. Confocal images of the engrafted bare islet to the iris with the β-cells expressing GCaMP3 (green) and vessels perfused with DyLight-Lectin (red). Green arrowhead points to the islet grafted to the iris (magenta arrowhead).
c) Intravital fluorescence imaging of transplanted microstructures 20.
   i. Image of an islet being vascularized from the iris.
   ii. In this image, vessels (white arrowheads) from the iris have sprouted and branched to vascularize the islet despite being far and in no direct contact (in a few tens of microns away) with the iris.
   iii. A fully vascularized islet is also shown in this image, which has been vascularized from the iris.
   iv. Although most samples are vascularized from the iris, this image shows a sample that is vascularized from both the iris and the cornea.

The physiological function of pancreatic islets in glycaemic control (sensing blood glucose and secreting hormones) requires vascularization. Consequently, revascularization of the transplanted islets is a critical step in ensuring their survival and function. To evaluate islet revascularization eight weeks post-transplantation, a fluorescent dye solution was injected into the tail vein of the mouse while monitoring islet fluorescence (Figure 4). DyLight conjugated Lectin was used as the neovascularization tracer to perfuse the vessels. Figure 4a depicts the fluorescent profile of a confined islet within a microstructure 20 before and after injecting the fluorescent dye. Movements related to animal respiration and injection can cause a loss of focus, leading to changes in signal (Figure 4a-iii). As a positive control for observing engraftment, bare islets were also transplanted bare islets, for which vascularization is stimulated by the proximity to the abundant vessels in the iris (Figure 4b). Capillary sprouts and new vessels were expected to start to form in transplanted islets within 2-4 days after transplantation.

During in vivo imaging, the perfusion of ten confined islets with the fluorescent dye was observed, confirming successful engraftment. Seven of these islets were vascularized from the iris, with the vascular connections originating from beneath the islets as well as from the surrounding region at close proximity (Figure 4c-i,ii,iii). Notably, there were vascular connections to the iris in the microstructure 20 that was not in direct contact with the iris (Figure 4c-ii). This microstructure 20 was only secured mechanically to the cornea, and upon dissecting the eye and breaking the delicate free-standing vasculature, the inventors found that the microstructure 20 was entirely free-floating.

Vascular network formation is an intricate process that involves endothelial cells, soluble growth factors and extracellular matrix (ECM) components and remodelling and is influenced by biochemical and biomechanical cues in the microenvironment. The results present compelling evidence of endothelial sprouting through the ECM-free microenvironment of aqueous humor. The aqueous humor is secreted at a rate of 1.5-3.0 µl.min⁻¹ and undergoes clearance approximately every 100 min, resulting in a complex signalling environment for pro-angiogenic mediators such as vascular endothelial growth factor (VEGF).

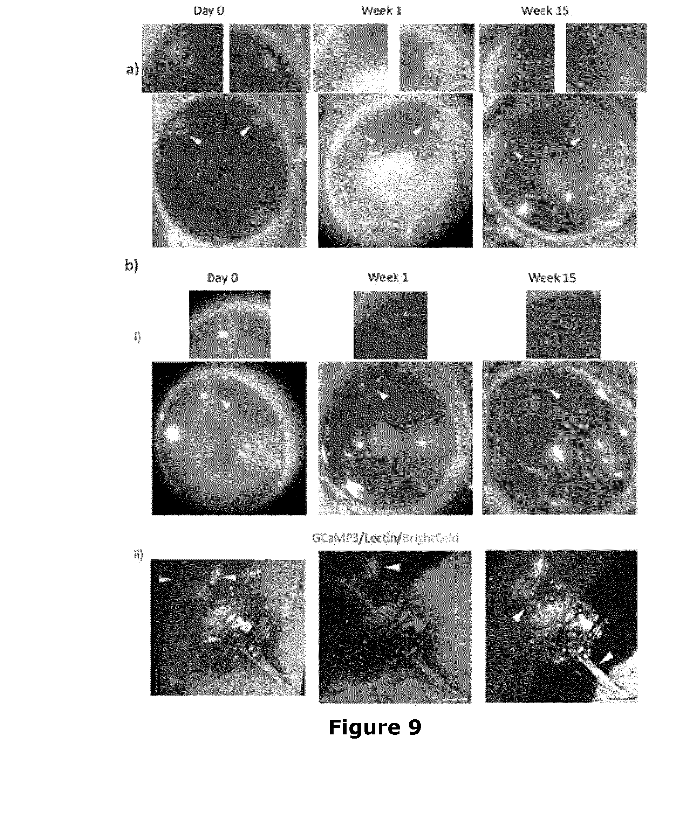

In the three remaining samples, the islets had vascular connections to the iris and cornea, as shown in Figure 4c-iv, representing a typical sample with a good transplantation procedure and a fully localized islet in the microstructure 20. However, in the second sample, a mechanical complication during transplantation led to cornea damage, resulting in subsequent haziness of the cornea, as shown in Figure 9a. This haziness suggested a condition known as cornea edema, which can occur when the cornea becomes excessively hydrated and gradually loses transparency, leading to corneal vascularization. Cornea edema can arise from various causes, including surgical interventions. Finally, in one of the 11 cases, the islet was trapped between the flap doors 26. Fifteen weeks after transplantation, we observed the islet outside the microstructure 20 and attached to the cornea, exhibiting morphological changes (Figure 9b-i). Furthermore, the islet had a vascular connection with the iris and cornea, as shown in Figure 9b-ii. In this sample, the inventors observed the same vascular extensions in the aqueous humor as in the previous samples that had solely vascular connection with the iris. In this sample, [Ca²⁺]ᵢ oscillations at week 15 post-transplantation were recorded.

Graft revascularization depends on various factors such as blood vessel density in the transplanted site, inflammation, oxygen tension and the intra-islet endothelial cells. Additionally, the VEGF produced by FBGCs helps recruit and maintain immature neovessels. Extracellular vimentin secreted by FBGCs may be pro-angiogenic and may have similar functionality as VEGF, including VEGF-receptor signalling. In the context of biohybrid engraftment, the role of FBR in forming and maintaining the vascular network can be advantageous.

### Conclusion

This study focused on investigating biohybrid microstructure transplantation and using TPP's benefits to fabricate microstructures 20 capable of delivering and fixating microtissues in the ACE. The fabrication procedure is simple and easily adjustable to fit various tissues and other modality elements. The flap doors 26 and confinement cage 24 make microtissue confinement free from using adhesive materials, such as hydrogels. This strategy can be used for various applications such as biohybrid sensors, microtissue delivery and defined tissue-tissue interaction studies.

The inventors utilized pancreatic islets expressing a genetically encoded Ca²⁺ indicator (GECI), GCaMP3, to evaluate their in vivo functionality over the transplantation period. GECIs are ideal for evaluating cell function in transplantable biohybrid devices since they enable longitudinal readouts and avoid the challenges associated with using fluorescent dyes in vivo, such as labeling and injection, concentration optimization, and experimental repeatability due to their short half-life. After evaluating the in vitro functionality of the microstructures 20, the inventors assessed the in vivo functionality and vascularization over 20 weeks using microscopy. Analysis of different microstructures 20 suggests that the dynamics of the iris is a critical factor for successful engraftment and that the localization is essential in the outcome. By targeting the iridocorneal angle, where the iris dynamics are minimal, a more stable geometry for the microstructure design is developed.

The invention represents a significant advance in the field of biohybrid structural engraftment in the ACE, providing compelling empirical evidence of successful integration. Moreover, the study highlights the potential for this unique site and strategy to be leveraged in a broad range of investigations requiring bio-microsystem integration with the host microenvironment. This includes studies enabling real-time measurements of physiological responses with high specificity and sensitivity, investigations into various microtissue-microtissue interactions, and exploration of ECM-free vascularization strategies. The inventors' finding that endothelial sprouting can occur in a dynamic fluid environment with low viscosity underscores the critical role that the physical and chemical properties of the microenvironment play in tissue regeneration and integration. By shedding light on these fundamental principles, the invention lays a foundation for future research to improve the efficacy of biohybrid structural integration and tissue engineering strategies.

### Experimental Section

### Mice for isolation and transplantation

The sensor expressing islet, expressing the fluorescent Ca²⁺ indicator protein (GCaMP3), was obtained from heterozygous donor mice (RIP-Cre:GcaMP3). The recipient mice, immunocompetent C57BL/6J, were obtained from the breeding colony at Karolinska Institutet, Stockholm, Sweden. Mice were kept on a 12h light/12h dark cycle. All experimental procedures and animal studies were carried out in strict accordance with the Karolinska Institutet's guidelines for the care and use of animals in research and were approved by the regional Animal Ethics Committee (number 1184-2021).

### Islets of Langerhans isolation and culture

Pancreatic islets were isolated from 8-10 weeks old adult wild-type and double heterozygous RIP-Cre:GCaMP3 male mice. In brief, mice were sacrificed by cervical dislocation, the abdomen was opened, and the common bile duct was clamped to confine the digestion buffer (ice cold collagenase P (1.5 mg.ml⁻¹, Roche) in the washing buffer containing Hanks' Balanced Salt Solution (HBSS, Gibco) supplemented with BSA (2.5 mg.ml⁻¹, Fisher) in HEPES (25 mM, Gibco), and pH adjusted to 7.4) in pancreas after bile duct injection. The perfused pancreas was separated from the abdomen, collected in a vial containing the washing buffer, and placed in a water bath (37°C, 25 min). To help with liberation from the tissue ECM, the digested solution was passed through an 18G steel needle and washed (twice, 5 min). Islets were handpicked under a stereomicroscope and incubated at standard conditions (37°C, 5% CO₂, in humidified air) in a medium containing RPMI-1640 (Gibco) supplemented with 10% FBS (Gibco), L-Glutamine (2mM, Gibco), Penicillin (100 IU.ml⁻¹, Gibco) and Streptomycin (100 µg.ml⁻¹, Gibco) until use.

### Microstructure fabrication and islet localization

Microstructures 20 were fabricated using a commercial TPP 3D printer (Nanoscribe) in a commercially available biocompatible photoresin (IP-Visio and IP-S, Nanoscribe). Photoresin was dispensed on indium tin oxide (ITO) coated glass, and the designs (designed in SolidWorks) were printed. Following TPP structuring, samples were developed in propylene glycol methyl ether acetate (PGMEA, Sigma-Aldrich) and washed with isopropanol (VWR Chemicals) for 25 min and 5 min, respectively. To prepare the structure for hosting an islet inside, samples were washed with culture media and centrifuged (2200 g for 5 min) to remove the air bubbles trapped inside the microstructure voids. Islets were picked and localized in the structures using a micromanipulator (Eppendorf, 5170). Borosilicate glass micropipettes were pulled and grinded to create microcapillaries or cannulas. Islets were localized inside the microstructures 20 one day after isolation. This delay gives the freshly isolated islets time to produce ECM around themselves and better tolerate the possible mechanical tensions during the localization procedure.

### In vitro [Ca²⁺]ᵢ imaging

Four samples consisting of a wild-type islet, biosensor (GCaMP3) expressing islet, microstructure 20 made of IP-S, and IP-Visio were prepared. Their fluorescence was examined using a laser scanning confocal microscope (TCS SP8, Leica) with gating of the emission signal to exclude reflection. To check the functionality of the localized islets before transplantation and absence of mechanical damage stemming from the localization procedure, in vitro confocal Ca²⁺ imaging was acquired. To verify the biocompatibility of IP-Visio on the islet's function, samples were localized in the structures, and a stimulation experiment, using 3 mM or 11 mM glucose, and KCl (25 mM), was performed after one week of culture using wide-field Ca²⁺ imaging. Microstructures 20 were first incubated in low glucose buffer medium (3 mM) for 1 h, and after 100 seconds of baseline Ca²⁺ signal recording, media was changed to a higher glucose concentration (11 mM) and then back to a low glucose medium. In the final step, KCl was added to depolarize the cells. The experiment was conducted at 37°C.

### Transplantation

Microstructures 20 were manually transplanted in the ACE of 8-10 weeks old C57BL/6J mice. Mice were anesthetized with isoflurane (2-2.5% (v/v), Baxter) using the UNIVENTOR 400 anaesthesia unit, and the head was supported and fixed under a stereomicroscope using a head holder (Narishige). Body temperature was maintained at 37°C ±0.5°C using a heating pad. A small incision was manually created on the cornea near the sclera using an 18-gauge steel needle. The microstructure 20 was carefully transplanted in the ACE using a glass capillary connected to a Hamilton syringe via polyethylene tubing. Microstructures 20 were injected in the ACE and positioned on the iris and at the iridocorneal angle. A drop of Viscotears (Novartis) was placed on the cornea to avoid desiccation. To visualize the state of the cornea after transplantation regarding any damage created during transplantation, eyes were visualized using an OCT scanner (OQ LabScope, Lumedica).

### In vivo [Ca²⁺]ᵢ imaging of the transplanted islets

Anaesthesia was induced and maintained by inhalation of vaporized isoflurane. Mice were fixed with the head holder and placed on the customized confocal laser scanning microscope (TCS SP5, Leica) stage. Viscotears was used as an immersion medium between the eye and the objective. Fluorescence emission from GCaMP3 was acquired at λ_{exc} = 488 nm and λₑₘᵢ = 500-550 nm. To visualize the microstructure 20 and its location in the ACE, a backscatter signal was also acquired by a 633 nm laser (λ_{exc} = 633 nm, λₑₘᵢ = 630-636 nm). In vivo Ca²⁺ imaging was performed on the day of transplantation and after one, two, 8, as well as 20 weeks post-transplantation.

### In vivo vascularization assessment

For in vivo vascularization, perfused blood vessels were identified by intravenous injection of DyLight 649 conjugated Lectin (1mg per mouse, Invitrogen) using microscopy.

### Immunohistochemistry

Animals were euthanized by cervical dislocation, and eyes were enucleated and fixed immediately (2 h) in formalin solution (10% neutral buffered, Sigma). Microstructures 20 were then retrieved from the ACE and permeabilized and blocked using a buffer (0.25% Triton X100 (Sigma-Aldrich) and 10% FBS in 1 x PBS). Samples stained for extracellular vimentin were not permeabilized and the buffer excluded Triton-X100. Samples were then incubated (overnight at 4°C) with a cocktail of primary antibodies diluted in a buffer (0.25% Triton-X100 and 5% FBS in 1 x PBS). The primary antibodies used in this study include anti-vimentin (Abcam ab92547, 1:100), anti-α-smooth muscle actin (a -SMA) (Invitrogen, 1:500), anti-F4/80 (Invitrogen, 1:100), anti-CD11b (BD Bioscience, 1:200), and anti-insulin (Dako 1:200). Samples were washed three times in blocking buffer and incubated with the secondary antibodies (1-2 h, room temperature). After three additional washes with PBS, nuclei were stained using DAPI (1:1000) or Hoechst (1:2000) and visualized using a laser scanning confocal microscope.

### Additional information

Figure 5 shows a cylinder microstructure design and islet localization procedure.
a) The CAD model of the cylinder microstructure 20. The structure contains micro-cavities for media transfer and giving access to endothelial cells sprouting and entering blood vessels, and a "flap door" feature for securing the islet inside.
b) Photographs showing islet localization in the microstructure 20.
   i. Comparison of islet and microstructure 20 before and after islet localization.
   ii. Islet is gently held by a cannula connected to a micromanipulator 34 and transferred to the microstructure 20.
   iii. Empty cannula is shown on top of the microstructure 20.
   iv. Islet held by the cannula is positioned on top of the microstructure 20 and is lowered to enter the microstructure 20 by opening the flap doors 26.

Figure 6 shows optical characterization of materials for 3D printing.
a) Images
   i. Confocal images of IP-S and IP-Visio structures (λ_{exc} = 480 nm, λₑₘᵢ = 492-544 nm).
   ii. Autofluorescence emission spectra of IP-S and IP-Visio plotted for different excitation wavelengths (480-640 nm). This wavelength range was chosen based on the fluorescence signals attributed to GCaMP3 and the fluorescent dyes used in the study.
b) Images
   i. Confocal fluorescence images of IP-S, GCaMP3 islet, wild-type islet, and IP-Visio.
   ii. Fluorescence emission intensity of the samples.
   iii. Ca²⁺ imaging of the GCaMP3 and wild-type islets in Figure 6b-i). (Scale bar = 100 µm).

IP-Visio and IP-S were investigated as the structural material. IP-Visio was selected due to its suitable optical properties (transparency and autofluorescence) and mechanical properties. The fluorescence emission spectral profiles of two commercially available photoresin used for cell studies, IP-S and IP-Visio, were analysed. Based on fluorescence spectrometry analysis, IP-Visio shows a considerably lower autofluorescence intensity between 480-640 nm (the spectral range for most fluorescence imaging) compared to IP-S (Figure 6a). Since the functionality of the transplants by optical imaging of the fluorescent Ca²⁺ indicator/sensor protein (GCaMP3) was evaluated, the difference between the fluorescence emission of the microstructure 20 and GCaMP3 is a critical factor. This difference was verified by comparing the autofluorescence emission intensity of IP-S, IP-Visio, GCaMP3 islet, and wild-type islet (not expressing GCaMP3). Results show that IP-Visio has a lower fluorescence compared to wild-type islets, GCaMP3 islet, and IP-S photoresin (Figure 6b). Results suggest that IP-Visio is tuned to meet the needs for biomedical imaging, including lower autofluorescence than wild-type islets, where intrinsic autofluorescence arises from the metabolic cofactors such as pyridine nucleotides and flavoproteins. The Young's modulus of IP-Visio is 1.31 GPa, making it a suitable material as a supportive structure during transplantation.

As for islet isolation, localization in the microstructure 20, and in vitro functionality, in β-cells, blood glucose-induced insulin release is regulated through a process that involves plasma membrane depolarization, Ca²⁺ influx to the cytosol through voltage-gated Ca²⁺ channels, increase in [Ca²⁺]ᵢ (cytoplasmic free Ca²⁺ concentration), and finally, triggering fusion of exocytotic insulin-containing vesicles with the plasma membrane and release of insulin into the blood (Figure 7a,b).

Figure 7 shows an overview of pancreatic islets and validation of biohybrid assembly and transplantation.
a) The pancreas has digestive (pancreatic acinar cells) and hormonal (pancreatic islet) functions. A pancreatic islet is a highly vascularized microtissue with different endocrine cells that regulate blood glucose homeostasis.
b) For experimental purposes, islets are liberated from the pancreas using enzymatic digestion, and they gradually lose their endothelial cell fraction in vitro. The islets used in this study are genetically engineered to express a green fluorescent Ca²⁺ sensor protein (GCaMP3) in their β-cell population. The sensor protein's fluorescence intensity is linked to [Ca²⁺]ᵢ, and can be optically quantified. High glucose levels control [Ca²⁺]ᵢ through various cellular compartments such as the endoplasmic reticulum, mitochondria, and the voltage-dependent and -independent Ca²⁺ channels.
c) Images
   i. Confocal fluorescent image of a microstructure 20 with an islet expressing GCaMP3.
   ii. Its [Ca²⁺]ᵢ oscillations in vitro in media containing 11mM glucose.
d) Glucose stimulation. To test IP-Visio's biocompatibility on the islet's function, glucose stimulation in GCaMP3 expressing islets after one week of culture in the microstructures 20 was performed. The GCaMP3 fluorescence signal was monitored after 1 hr incubation at 3mM glucose. The microstructure 20 was perfused with 3 mM (~ 100 s), 11 mM Glucose (~800 s), 3 mM glucose (~1100 s), KCl (~1200 s), and 3 mM glucose (~1500 s).

Figure 8 shows vascularization of cylinder microstructures 20.
a) Images
   i. Photograph of transplanted eyes showing the localization and the orientation of the transplanted microstructure 20 after one and two weeks of transplantation.
   ii. Schematic representation of the microstructure movement due to the pupillary dynamics that prevent successful vascularization. Δx represents displacement.
b) Intravital fluorescence images of cylinder microstructures 20 at different time points after transplantation. Blood vessels were perfused by the fluorescent dye, DyLight- Lectin (red), administered through tail vein injection. Wherever applied, arrowheads denote the cornea (cyan), microstructure 20 (yellow), and iris (magenta).
   i. Microstructure 20 experiencing movements related to iris dynamics and failed islet vascularization.
   ii. Microstructure 20 fixed at the angle and experiencing a more stable condition in favour of vascularization. Red denotes perfusion of the dye through the vasculature. Sometimes, here observed in images taken at week 4 and 10 months, the corneal endothelium also uptakes and stains with the injected dye. (Scale bar = 100 µm).

Figure 9 shows mouse eyes after transplantation. Arrowheads denote cornea (cyan), microstructure 20 (yellow), iris (magenta), and vessels (white).
a) Photograph image of an eye that experienced a complication during transplantation, and the cornea eventually turned cloudy. This eye was used as a recipient for an experimenting transplantation procedure and received two microstructures 20.
b) Images
   i. Photograph image of an eye transplanted with a loosely secured islet in the microcarrier. The islet eventually escaped from the microstructure 20 and a residue remained attached to the cornea.
   ii. Confocal image of the transplanted sample at week 15 post-transplantation. Vascularization from the iris and the cornea to the microcarrier is observed.

The recited numerical values, e.g. of parameters and dimensions, are non-limiting and are intended to illustrate the working of the invention.

The listing or discussion of an apparently prior-published document or apparently prior-published information in this specification should not necessarily be taken as an acknowledgement that the document or information is part of the state of the art or is common general knowledge.

Preferences and options for a given aspect, feature or parameter of the invention should, unless the context indicates otherwise, be regarded as having been disclosed in combination with any and all preferences and options for all other aspects, features and parameters of the invention.

## Claims

1. An implantable medical device comprising a modality delivery structure, wherein the modality delivery structure includes:
a confinement volume for confining a modality element, wherein the confinement volume is configured to, in use, enable at least partial interaction of the confined modality element with the environment outside of the confinement volume;
an access element that is mechanically openable and closeable to control access into the confinement volume; and
an anchor portion for mechanical anchoring within a human or animal body or body part.

2. An implantable medical device according to Claim 1 wherein the confinement volume is configured to, in use, enable at least partial vascularisation of the confined modality element with the environment outside of the confinement volume.

3. An implantable medical device according to Claim 1 or Claim 2 wherein the confinement volume is configured to, in use, enable at least partial release of the confined modality element out of the confinement volume.

4. An implantable medical device according to any one of the preceding claims wherein the confinement volume is a confinement cage.

5. An implantable medical device according to any one of the preceding claims wherein the access element includes at least one flap member that is mechanically openable and closeable to control access into the confinement volume, preferably wherein the access element includes a pair of opposing flap members that is mechanically openable and closeable to control access into the confinement volume.

6. An implantable medical device according to any one of the preceding claims wherein the access element is movable to a first position to permit access into the confinement volume, and the access element is movable to a second position to restrict access into the confinement volume.

7. An implantable medical device according to any one of the preceding claims wherein the access element is configured to automatically return to the second position from the first position upon removal of a force applied on the access element, and/or wherein the access element is made of a resilient material.

8. An implantable medical device according to any one of the preceding claims wherein the anchor portion is configured as an anchor portion for sutureless mechanical anchoring within the human or animal body or body part.

9. An implantable medical device according to any one of the preceding claims wherein the anchor portion is configured as a non-penetrative anchor portion for mechanical anchoring within the human or animal body or body part.

10. An implantable medical device according to any one of the preceding claims wherein the anchor portion and/or the modality delivery structure is shaped as a wedge.

11. An implantable medical device according to any one of the preceding claims wherein the confinement volume is arranged between first and second layers that are angled with respect to each other, preferably wherein the first and second layers are arranged at an acute angle with respect to each other.

12. An implantable medical device according to any one of the preceding claims wherein the implantable medical device is an intraocular implant.

13. An implantable medical device according to Claim 12 wherein the anchor portion is configured for mechanical anchoring within an iridocorneal angle of the eye, preferably wherein the anchor portion is shaped to correspond to geometry of the iridocorneal angle of the eye.

14. An implantable medical device according to any one of the preceding claims wherein the modality element includes tissue, a pancreatic islet, a therapeutic modality element, a drug and/or a medicament.

15. A method of configuring an implantable medical device according to any one of the preceding claims, wherein the method comprises the steps of opening the access element and inserting a modality element into the confinement volume.
